# EUROPÄISCHE PATENTSCHRIFT

(11) **EP 1 926 499 B1**
(45) Veröffentlichungstag und Bekanntmachung des Hinweises auf die Patenterteilung: **22.01.2014**
(21) Anmeldenummer: 06805305.7
(22) Anmeldetag: 18.09.2006
(51) Int. Cl.: A61K 47/48, A61K 31/685

(54) **VERWENDUNG VON THERAPEUTISCH WIRKSAMEN LIPIDEN NEBST VERFAHREN ZUR HERSTELLUNG VON ORGAN-/GEWEBE- SPEZIFISCHEN THERAPEUTISCH WIRKSAMEN LIPIDEN**
USE OF THERAPEUTICALLY ACTIVE LIPIDS TOGETHER WITH METHOD OF PRODUCING ORGAN/TISSUE-SPECIFIC THERAPEUTICALLY ACTIVE LIPIDS
UTILISATION DE LIPIDES A EFFET THERAPEUTIQUE ET PROCEDE DE FABRICATION DE LIPIDES A EFFET THERAPEUTIQUE SPECIFIQUES AUX ORGANES/TISSUS

(30) Priorität: 21.09.2005 DE 102005045152
(43) Veröffentlichungstag der Anmeldung: 04.06.2008
(73) Patentinhaber: PAT GmbH, 81375 München (DE)
(72) Erfinder: Stremmel, Wolfgang, 69120 Heidelberg (DE)
(74) Vertreter: Ullrich & Naumann
(86) Internationale Anmeldenummer: PCT/DE2006/001651
(87) Internationale Veröffentlichungsnummer: WO 2007/033654

(56) Entgegenhaltungen:
- WO-A1-2004/037275
- WO-A2-01/51003
- MILKIEWICZ P ET AL: "Hepatoprotection with tauroursodeoxycholate and beta muricholate against taurolithocholate induced cholestasis: involvement of signal transduction pathways.", GUT JUL 2002 LNKD- PUBMED:12077103, vol. 51, no. 1, July 2002 (2002-07), pages 113-119, ISSN: 0017-5749
- WALEE CHAMULITRAT ET AL: 'Bile salt-phospholipid conjugate ursodeoxycholyl lysophosphatidylethanolamide as a hepatoprotective agent' HEPATOLOGY Bd. 50, Nr. 1, 01 Juli 2009, Seiten 143 - 154, XP055004384 DOI: 10.1002/hep.22955 ISSN: 0270-9139

## Beschreibung

Die vorliegende Erfindung betrifft ein Lyso-Phospholipid, das kovalent an Gallensäuren oder Asialoglycoproteine gebunden ist zur Verwendung für die Behandlung entzündlicher Lebererkrankungen. Des Weiteren betrifft die vorliegende Erfindung ein Verfahren zur Herstellung von Leber-spezifischen therapeutisch wirksamen Lipiden zur Behandlung entzündlicher Lebererkrankungen, dadurch gekennzeichnet, dass LysoPE an die zu einem Ester umgewandelte Carboxylgruppe von Urso-DOCA zu einer Lyso-PE-DOCA Verbindung gekoppelt wird.

Bestimmte therapeutisch wertvolle Lipide sind pro- bzw. antiinflammatorisch wirksam (ANES und Mitarbeiter - Nat. Cell. Biol. 2003; 6: 793-802). WO 01/51003 offenbart Lipid-Konjugate mit Gallensäuren zur medizinischen Verwendung. Bereits in *in-vitro*-Modellen an Makrophagenmodellen und Phagosomen konnte eine therapeutische Wirkung von Phosphatidylcholin (PC) und Lyso-Phosphatidylcholin (LysoPC) bei Anreicherung dieser Lipide in den betroffenen Zellen auf entzündliche Aktivitäten oder einen gestörten Metabolismus nachgewiesen werden (Anes und Mitarbeiter - Nat. Cell. Biol. 2003; 5: 793-802). Auch im Tiermodell ist eine therapeutische Wirkung von PC schon exemplarisch gezeigt worden, wobei die topische Applikation des antientzündlich wirkenden Phosphatidylcholins in Rattenmodellen die Colonmucosa vor einer durch Essigsäure- und Trinitrobenzsulfonsäure-induzierten Colitis schützt (FABIAN et al. -Digestion 1992; 53: 35-44; MOURELLE et al. - Gastroenterology 1996; 110: 1903-7). Auch beim Menschen wurde dieses Prinzip bei der Behandlung der häufigen chronisch entzündlichen Darmerkrankung Colitis ulcerosa angewandt. Dabei konnte gezeigt werden, dass die orale Gabe von Phosphatidylcholin in retardient verpackter Form mit Freisetzung im unteren Dünndarm und Dickdarm die entzündliche Aktivität der Colitis ulcerosa signifikant unterdrückt (STREMMEL et al. - Gut 2005; 54: 966-997, europäisches Patent 1 105 141 B1). Im Vergleich zu einer mit Placebo behandelten Kontrollgruppe konnte bei 90 % der mit dem PC behandelten Patienten eine im Mittel 70 %ige Verbesserung der klinischen Aktivität erreicht werden. Innerhalb von 3 Monaten erreichte sogar über die Hälfte der Patienten eine klinische Remission. Gleichzeitig besserte sich der endoskopische Befund und die Histologie im unteren Dünndarm und Dickdarm sowie die Lebensqualität der Patienten.

Aufgrund der vorliegenden Ergebnisse ist es wahrscheinlich, dass auch mit Entzündungen assoziierte Erkrankungen In anderen Zellen, Geweben und Organen neben dem Dünndarm und Dickdarm mit antiinflammatorisch wirksamen Lipiden zu behandeln sind. Allerdings ist dabei nicht zu erwarten, dass die systemische unselektive Gabe von zum Beispiel Phosphatidylcholin die notwendigen antiinflammatorischen Wirkspiegel vor Ort erreicht, weshalb eine lokale Applikation erforderlich wäre. So können zum Beispiel bei lokalen, inflammatorisch bedingten Zuständen antiinflammatorische Lipide als solche lokal appliziert werden, wie z.B. bei der topischen Anwendung auf entzündeten Hautareaten, bei der Instillation in Gelenkhöhlen bei Arthritis, bei der inhalation geeigneter Präparationen in das Bronchialsystem zur Behandlung von Entzündungen der Lunge oder bei der Instillation von Lipidsuspensionen in den Gastrointestinaltrakt, z.B. den Ösophagus, den Magen, das Duodenum und das Rektum.

Die lokale Applikation In parenchymatösen Organen wie Leber, Herz und Gehirn gestaltet sich dabei schwieriger. Diese könnte zwar zur Erreichung einer hohen lokalen Konzentration in diesen Organen als lokale Infusion von therapeutisch wirksamen Lipidsuspensionen oder durch die Anwendung reversibler Embolisationstechniken erfolgen, welche aber im Hinblick auf die Dauer der Infusion und die Risiken, die bei der Anwendung der Embolisationtechnik zu befürchten sind, keine wirklichen Alternativen zu der lokalen Applikation darstellen.

Der vorliegenden Erfindung liegt daher die Aufgabe zugrunde, eine Verwendung von therapeutisch wirksamen Lipiden zur Organ-/Gewebe-spezifischen Anreicherung für die Behandlung entzündlicher, ischämischer oder degenerativer Erkrankungen und/oder zur Stimulation einer Regeneration der eingangs genannten Art für sämtliche Organe, Gewebe und Zellen unter Ausschließung von Risiken und langandauernder Infusionen anzugeben, die zu einer therapeutisch wirksamen Konzentration der Lipide in den Organen, Geweben und Zellen führt.

Erfindungsgemäß wird die voranstehende Aufgabe mit den Merkmalen der Patentansprüche 1, 7 und 8 gelöst Danach ist die Verwendung dieser therapeutisch wirksamen Lipide derart ausgestaltet, dass die Lipide bei Applikation an Gallensäuren oder Asialoglycoproteine als Trägermoleküle gebunden sind, für die zellspezifische Aufnahmesysteme In den Zellen der Organe und/oder der Gewebe existieren.

Des Weiteren ist die obige Aufgabe mit den Merkmalen des Patentanspruchs 6 gelöst. Danach ist ein Verfahren zur Herstellung von Organ-/Gewebe-spezifischen therapeutisch wirksamen Lipiden der eingangs genannten Art derart ausgestaltet, dass Lyso-Phosphatidyläthanolamin (LysoPE) an die zu einem Ester umgewandelte Carboxylgruppe von Ursodeoxycholat (UrsoDOCA) zu einer LysoPE-DOCA-Verbindung gekoppelt wird.

In erfindungsgemäßer Weise ist erkannt worden, dass durch die Verwendung von an Trägermoleküle gekoppelte Lipide eine spezifische Anreicherung dieser Lipide auch in parenchymatösen Organen unter Ausschluss von Embolisations- oder Infusionstechniken erzielbar ist, wenn für die Trägermoleküle Zell-, Gewebe- oder Organ-spezifische Aufnahmesysteme existieren. Des Weiteren Ist erkannt worden, dass die Zellen komplexe Lipide, wie zum Beispiel Phosphatidylcholin nicht aufnehmen können und daher Lyso-Phospholipide, die nachweislich in die Zellen aufgenommen werden, an die Trägermoleküle gekoppelt werden. Entsprechend ist durch dieses zellspezifische "Targeting" eine Aufnahme der therapeutisch wirksamen Lipide selektiv in Zellen, Gewebe oder Organe bis zu einer therapeutisch wirksamen Konzentration möglich. So kann zum Beispiel eine therapeutisch wirksame Konzentration an Lipiden in Leberzellen, die entzündliche Veränderungen aufweisen, mit den nach dem Verfahren nach Anspruch 6 hergestellten Lipidverbindungen erreicht werden.

In besonders vorteilhafter Weise wirken die therapeutisch wirksamen Lipide antiinflammatorisch oder proinflammatorisch. Ist die durch die Trägermoleküle vermittelte spezifische Anreicherung der Lipide oder aber auch die durch die direkte. die inhalative oder die tropfenweise Applikation der therapeutisch wirksamen Lipide, wie zum Beispiel Phosphatidylcholin oder Lyso-Phosphatidylcholin (LysoPC), in die Zellen, Gewebe oder Organe gelungen, kann sich deren antientzündliche Wirkung entfalten. Neben der reinen Substitution eventuell fehlender Lipide wird der therapeutische Effekt zusätzlich durch eine intrinsische antiinflammatorische Aktivität der Lipide durch Einfluss auf Mediatoren und Signalübertragungswege vermittelt. Auf diese Weise sind beispielsweise Entzündungsreaktionen, die als physiologische Antwort, z.B. auf mechanische/degenerative, Erreger-bedingte, Ischämische, freie Radikal-induzierte, immunologisch vermittelte oder durch Medikamente, Drogen, Chemikalien oder Strahlen bedingte Schädigungs-mechanismen hervorgerufen werden, mit den antiinflammatorisch wirksamen Lipide behandelbar. Auch ist bei der Aufnahme dieser Lipide In Makrophagen oder in andere Zellen des Abwehr- und Immunsystems eine immunsuppressive Wirkung möglich.

Die Verwendung proinflammatorischer Lipide ist insbesondere bei einer gewollten Regeneration indiziert. Da eine Entzündung (vermittelt durch Mediatoren, wie z.B. IL6 und TNFα) in der Folge oft eine Regeneration induziert, kann durch therapeutisch eingesetzte proinflammatorische Lipide - wiederum topisch appliziert - die Regeneration, Reparatur und Hyperplasie von Zellen, Geweben und Organen propagiert werden. Beispielsweise kann die regenerative Kapazität der Leber genutzt werden, um danach größere Leberteilresektionen bei Tumorbefall (In anderen Lebersegmenten) zu ermöglichen. In besonders vorteilhafter Weise ist die Verwendung von proinflammatorischen Lipiden In Organen bevorzugt, bei denen die Zellmasse durch chronische Erkrankung oder akzidentellen/OP-induzierten Verlust eine kritische Genese erreicht hat.

Besonders vorteilhaft ist die Verwendung von proinflammatorisch wirkenden Lipide vor geplanter ausgedehnter Leberteilresektion, z.B. um eine Lebermetastase zu entfernen, um genügend Leberzellmasse in der Restleber zu induzierten. Dazu wird das proinflammatorische Lipid vor der Operation selektiv in die später noch verbleibende Restleber instilliert.

Darüber hinaus kann der proinflammatorische Effekt die Immunstimulation in malignen Zellen fördern und damit für die Antitumortherapie (z.B. Induktion von Apoptose) genutzt werden.

Des Weiteren ist die Verwendung von im zu behandeinden Organismus natürlich vorkommenden Liganden als Trägermoleküle vorteilhaft, an die die Lipide kovalent gebunden sind. Die Liganden werden entsprechend von Zell-spezifischen Rezeptoren der basolateral membranassoziierten Transportersysteme erkannt, über die die Lipide in die Zelle geschleust werden. So sind die Lipide zum Beispiel kovalent an Gallensäuren oder Asialoglycoproteine als Liganden für eine Hepatozyten-spezifische Aufnahme gebunden. Als ein Hepatozytenspezifisches Transportersystem ist besonders das Na⁺/Taurocholat cotransportierende Polypeptid (NTCP-Transporterprotein) zu nennen, über das mehr als 90 % der Gallensäuren In die Leberzellen eingeschleust werden.

Im Hinblick auf die Entfaltung der antiinflammatorischen oder proinflammatorischen Wirkung in der Zelle sind die Lipide, wie zum Beispiel Lyso-Phosphatidylcholin (LysoPC) oder Lyso-Phosphatidyläthanolamin (LysoPE) nach Aufnahme in den cytoplasmatischen Raum der Zelle von den Liganden freisetzbar. Dies erfolgt beispielsweise durch Hydrolyse im Cytoplasma. Wird, wie in dem oben genannten Fall, Lyso-Phosphatidyläthanolamin in die Zelle eingeschleust, erfolgt nach der Freisetzung vom Liganden eine enzymatische Umwandlung des LysoPE zu dem antiinflammatorisch wirkenden PC.

Ganz allgemein stehen bei der Verwendung der therapeutisch wirksamen Lipide für die Verabreichung sämtliche Applikationswege zur Verfügung, wie zum Beispiel die direkte, die Inhalative, die tropfenweise (Instillation), die orale, die intravenöse oder die intraperitoneale Applikation. Dabei eignet sich, wie oben beschrieben, bei der Verwendung anti-entzündlicher Lipide insbesondere die direkte Applikation zur Behandlung entzündungsgeschädigter Haut oder die Injektion in Gelenke oder Liquorräume. Die orale, intravenöse oder intraperitoneale Applikation ist besonders bei der selektiven Anreicherung von therapeutisch wirksamen Lipiden in parenchymatösen Organen, Geweben und in Zellen mit entzündlichen Veränderungen angezeigt. Dabei eignet sich insbesondere die intravenöse Applikation der an die Trägermoleküle gebundenen Lipide, da diese über die Blutbahn direkt an die entsprechenden Organe. Gewebe oder Zellen gelangen, ohne zuvor, wie bei der oralen Verabreichung, die Passage über die Darmwand zu machen, was die Gefahr der vollständigen Aufnahme der Lipide durch die Darmzellen in sich birgt.

Insgesamt bietet die hier beschriebene lipidbasierte Therapie insgesamt eine gute klinische Wirksamkeit bei geringem bis fehlendem Nebenwirkungsprofil, da es sich um natürliche im Organismus vorkommende nicht immunogene Lipide handelt. Daraus ergibt sich die Möglichkeit, lokal hohe Dosen dieser Lipide zu verabreichen, wobei die systemischen Konzentrationen im Bereich physiologischer Grenzen bleiben.

Es gibt nun verschiedene Möglichkeiten, die Lehre der vorliegenden Erfindung In vorteilhafter Weise auszugestalten und welterzubliden. Dazu ist einerseits auf die nachgeordneten Ansprüche, andererseits auf die nachfolgende Erläuterüng eines bevorzugten Ausführungsbeispiels zu verweisen, das das beanspruchte Verfahren zur Herstellung von Organ-/Gewebe-spezifischen therapeutisch wirksamen Lipiden erläutert.

### Beispiel

Exemplarische Anreicherung von antiinflammatorischen Phospholipiden in Hepatozyten, um eine Leberentzündung zu unterdrücken.

Komplexe Lipide wie Phosphatidylcholin (PC) können als solche nicht in Zellen aufgenommen werden. Die Aufnahme erfolgt in Form von Lyso-Phospholipiden, die kovalent an einen Liganden, wie zum Beispiel an eine Gallensäure oder ein Asialoglycoprotein gebunden sind, die so über Gallensäuretransporter (beispielsweise NTCP) an der basolateralen Plasmamembran der Hepatozyten in den cytoplasmatischen Raum der Zellen aufgenommen werden. Dazu kann zum Beispiel Lyso-Phosphatidylcholin (LysoPC) an Gallensäuren kovalent gebunden werden, beispielsweise an Deoxycholat (DOCA). Allerdings ist die Synthese einer gekoppelten Lysophospholipid-DOCA-Verbindung mit Lyso-Phosphatidyl-äthanolamin (LysoPE) chemisch sehr viel günstiger. Folgende Reaktion kann beispielsweise realisiert werden: Zunächst wird die Carboxylgruppe des UrsoDOCA durch ein Carbodiimid zu einem aktivierten Ester umgewandelt. Anschließend erfolgt die Kopplung an LPE. Diese Kopfgruppenkonjugation erlaubt die Passage der LysoPE-DOCA-Verbindung durch Gallensäurentransporter in die Leberzellan. Dort kann durch hydrolytische Spaltung LysoPE wieder freigesetzt werden, welches dann enzymatisch zu dem antiinflammatorischen PC umgewandelt wird.

Im Vergleich kann eine Acylketten-Konjugation durchgeführt werden, bei der DOCA an die Acylseitenkette der Sn-1 oder Sn-2 Position des PC gekoppelt wird. Dabei muss nach Exposition mit Phospholipase A2 oder A1 die Sn-1 oder Sn-2 Seltengruppe zu einer Alkoholverbindung umgewandelt werden. Diese wird mit einem Intermediat aus der Reaktion zwischen Pyridin und dem aktivierten DOCA-Ester sowie Carbodiimid zur Kopplung gebracht.

Das folgende Reaktlonsschema veranschaulicht den oben beschriebenen Reaktionsablauf:

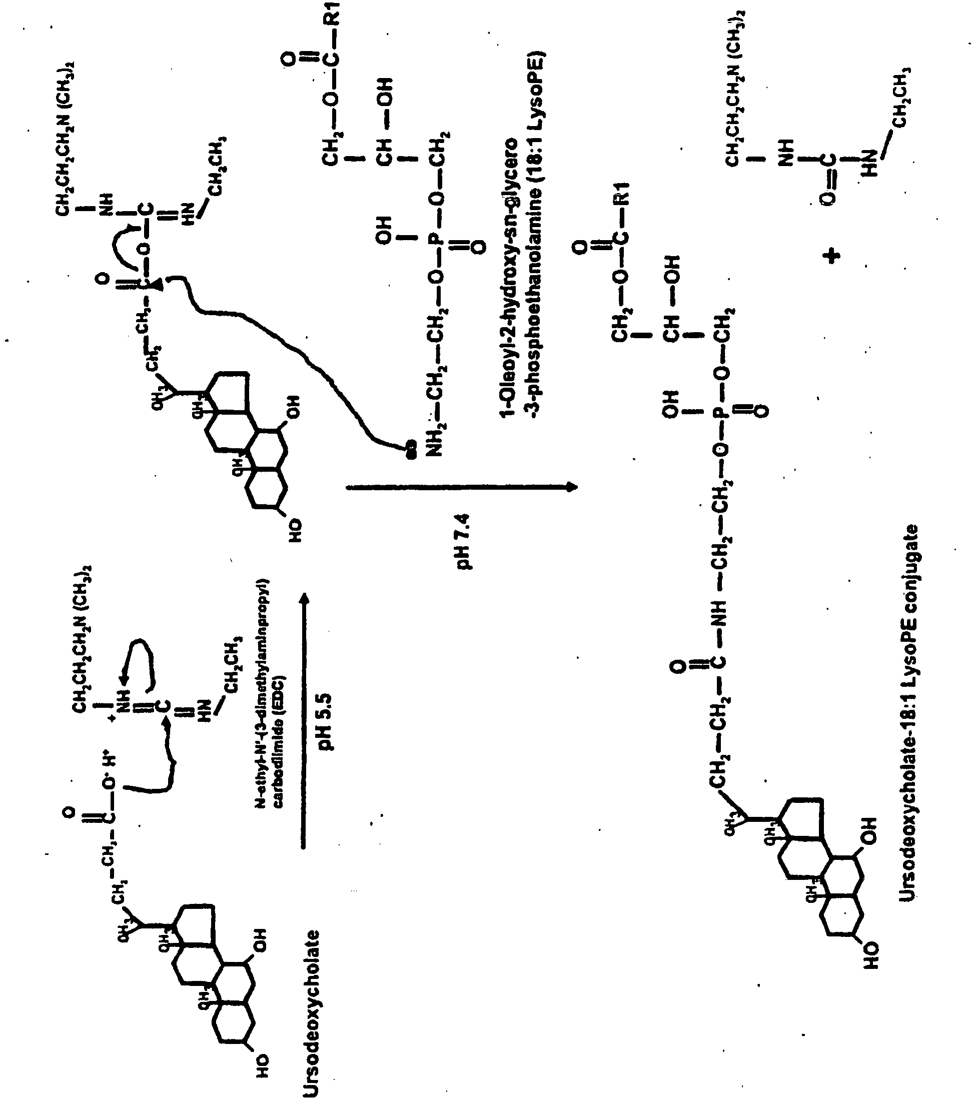

Zur Überprüfung der selektiven Anreicherung der LysoPE-DOCA-Verbindung wurden HepG2-Zellen (Hepatozyten) mit einem fluoreszensmarkierten Ursodeoxycholat-1-palmitoyl, 2-NBD-PE-Konjugat (bei 50 uM) für 10 min bei 37° C Inkubiert, gewaschen und danach mit 5 % Rinder-Serum-Albumin behandelt, um das Konjugat von der äußeren Plasmamembran der Zellen zu entfernen. Danach erfolgte die mikroskopische Betrachtung der Zellen im Phasenkontrast und im Fluoreszenslicht.

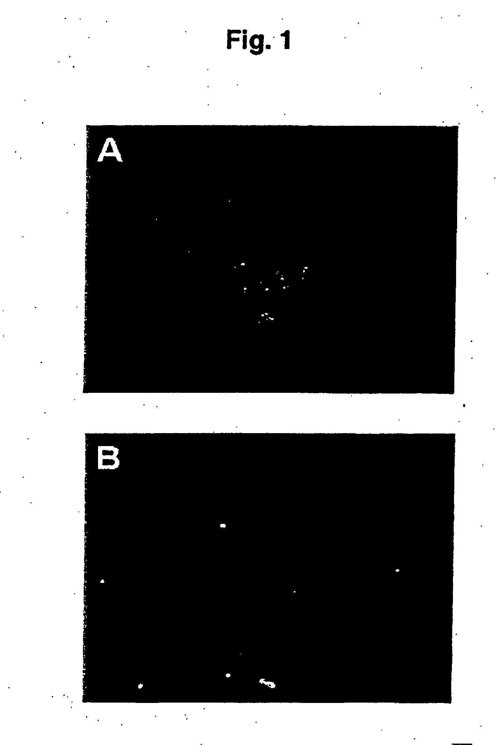

Fig. 1 zeigt in Bild **A** die Lichtmikroskopische Betrachtung von HepG2-Zellen nach Inkubation mit Ursodeoxycholat-1-palmitoyl, 2-NBD-PE-Konjugat im Phasenkontrast und Bild **B** zeigt die Fluoreszensmikroskopische Betrachtung der in A gezeigten Zellen.

Fig. 1 zeigt in Bild A und Bild B HepG2-Zellen (Hepatozyten) nach Inkubation mit einem erfindungsgemäßen therapeutisch wirksamen Lipid (Ursodeoxycholat-1-palmitoyl, 2-NBD-PE-Konjugat), welches zur Darstellung der Anreicherung in den Zellen mit einem Fluoreszensmarker markiert ist. Im Gegensatz zu den unter A im Phasenkontrast betrachteten HepG2-Zellen, zeigen die gleichen Zellen unter Fluoreszensmikroskopischer Betrachtung eine intensive gelb-grüne Färbung im Cytoplasma aber nicht im Kern der Zelle.

## Patentansprüche

1. Lyso-Phospholipid, das kovalent an Gallensäuren oder Asialoglycoproteine gebunden ist, zur Verwendung für die Behandlung entzündlicherisch, ischämischer oder degenerativer Lebererkrankungen.

2. Lyso-Phospholipid zur Verwendung nach Anspruch 1, wobei das Lyso-Phospholipid Lyso-Phosphatidylcholin oder Lyso-Phosphatidyläthanolamin ist.

3. Lyso-Phospholipid zur Verwendung nach Anspruch 1, wobei die Gallensäure Ursodeoxycholat oder Deoxycholat ist.

4. Lyso-Phospholipid zur Verwendung nach einem der Ansprüche 1 bis 3, **dadurch gekennzeichnet, dass** das Lyso-Phospholipid nach Aufnahme in die Zellen vom Ligand freisetzbar ist.

5. Lyso-Phospholipid zur Verwendung nach einem der Ansprüche 1 bis 4, das direkt, inhalativ, tropfenweise, oral, intravenös oder intraperitoneal verabreicht wird.

6. Verfahren zur Herstellung von Leber-spezifischen therapeutisch wirksamen Lipiden zur Behandlung entzündlicher Lebererkrankungen, **dadurch gekennzeichnet, dass** Lyso-Phosphatidyläthanolamin (LysoPE) an die zu einem Ester umgewandelte Carboxylgruppe von Ursodeoxycholat (UrsoDOCA) zu einer LysoPE-DOCA-Verbindung gekoppelt wird.

7. LysoPE-DOCA-Verbindung erhältlich nach dem Verfahren nach Anspruch 6.

8. Pharmazeutische Zusammensatzung, enthaltend die LysoPE-DOCA-Verbindung nach Anspruch 7.

## Claims

1. Lysophospholipid which is covalently bonded to gallic acids or asialoglycoproteins, for use for the treatment of inflammatory, ischaemic or degenerative liver diseases.

2. Lysophospholipid for use according to claim 1, the lysophospholipid being lysophosphatidylcholine or lysophosphatidylethanolamine.

3. Lysophospholipid for use according to claim 1, the gallic acid being ursodeoxycholate or deoxycholate

4. Lysophospholipid for use according to any one of claims 1 to 3, **characterised in that** the lysophospholipid can be released after being absorbed in the cells of the ligand.

5. Lysophospholipid for use according to any one of claims 1 to 4, which is administered directly, in an inhalational manner, drop-by-drop, orally, intravenously or in an intraperitoneal manner

6. Method for the production of hepatospecific therapeutically effective lipids for the treatment of inflammatory liver diseases, **characterised in that** lysophosphatidylethanolamine (LysoPE) is linked to the carboxyl group of ursodeoxycholate (UrsoDOCA), which group has been converted into an ester, to form a LysoPE-DOCA compound

7. LysoPE-DOCA compound which can be obtained according to the method in accordance with claim 6.

8. Pharmaceutical composition containing the LysoPE-DOCA compound in accordance with claim 7.

## Revendications

1. Lyso-phospholipide qui est lié par covalence à des acides galliques ou des asialoglycoprotéines, destiné à être utilisé pour le traitement d'affections hépatiques inflammatoires, ischémiques ou dégénératives.

2. Lyso-phospholipide destiné à être utilisé selon la revendication 1, le lysophospholipide étant de la lyso-phosphatidylcholine ou de la lysophosphatidyléthanolamine.

3. Lyso-phospholipide destiné à être utilisé selon la revendication 1, l'acide gallique étant de l'ursodésoxycholate ou du désoxycholate.

4. Lyso-phospholipide destiné à être utilisé selon l'une quelconque des revendications 1 à 3, **caractérisé en ce que** le lyso-phospholipide peut être libéré du ligand après absorption dans les cellules.

5. Lyso-phospholipide destiné à être utilisé selon l'une quelconque des revendications 1 à 4, qui est administré directement, par inhalation, goutte à goutte, oralement, par voie intraveineuse, ou par voie intrapéritonéale.

6. Procédé de préparation de lipides à efficacité spécifique de thérapie hépatique pour le traitement d'affections hépatiques inflammatoires, **caractérisé en ce que** de la lyso-phosphatidyléthanolamine (LysoPE) est attachée au groupe carboxyle, converti en un ester, de l'ursodésoxycholate (UrsoDOCA) pour donner un composé LysoPE-DOCA.

7. Composé LysoPE-DOCA, réalisable d'après le procédé selon la revendication 6.

8. Composition pharmaceutique contenant le composé LysoPE-DOCA selon la revendication 7.
